# EUROPEAN PATENT SPECIFICATION

(11) **EP 1 148 848 B1**
(45) Date of publication and mention of the grant of the patent: **26.08.2009**
(21) Application number: 00901459.8
(22) Date of filing: 03.02.2000
(51) Int. Cl.: A61F 2/44

(54) **END MEMBER FOR A BONE FUSION IMPLANT**
ENDSTÜCK FÜR KNOCHEN-FUSIONSIMPLANTAT
ELEMENT D'EXTREMITE POUR IMPLANT DE SOUDURE OSSEUSE

(30) Priority: 04.02.1999 US 118806 P
(43) Date of publication of application: 31.10.2001
(73) Proprietor: Synthes GmbH, 4436 Oberdorf (CH)
(72) Inventor: MESSERLI, Dominique, D., West Chester, PA 19380 (US); PAUL, David, C., Phoenixville, PA 19460 (US)
(74) Representative: Lusuardi, Werther
(86) International application number: PCT/CH2000/000060
(87) International publication number: WO 2000/045751

(56) References cited:
- WO-A-98/29047
- WO-A-99/32055
- DE-A- 19 509 317
- DE-A- 19 622 827
- GB-A- 2 338 652
- US-A- 5 702 451

## Description

The present invention relates to a device for fusion of bone, particularly vertebrae, and in particular to an end member for an implant for fusing bone or particularly vertebrae as defined in the preamble of the independent claims 1, 37 and 38.

The treatment and management of bone defects remains one of the most challenging aspects of orthopaedics. Bone defects occur in a wide variety of clinical situations. Restoring natural anatomical length and shape of any long bone with a bone defect is problematic. Additionally, whenever a vertebra has to be removed, it is necessary to insert a vertebral spacer to restore the natural length and curvature of the spine as well as to promote bone fusion. Restoring spinal anatomy and stability and promoting bone fusion are even more difficult in a corpectomy, a surgical procedure in which a section of one or several vertebrae is removed.

Many different implants have been developed for use with bone defects. So-called mesh implants" have shown to be particularly effective. One such implant is the SynMesh™ available from Synthes (USA) of Paoli, Pennsylvania. The SynMesh™ is a titanium mesh cylinder which is provided with a plurality of uniformly-spaced apertures of uniform size distributed on its surface. In order to address the different clinical situations in which bone defects arise, the SynMesh™ is available in a variety of lengths and diameters. Commercially available allografts for treating bone defects have recently become available.

Although the clinical results of mesh and allograft implants have generally been positive, one possible clinical complication is subsidence of the implant. Excessive sinking of the ends of the implant into the bone with which they contact is especially troublesome whenever the bone is extremely porous. From both a mechanical and physiological point-of-view, it would also be advantageous to increase the surface contact area between the implant and bone. U.S. Patent No. 5,702,451 to Biedermann et al. discloses end rings that attempt to address these concerns. However, the end rings disclosed have no provisions for holding, insertion, and distraction instruments. Thus, implantation can be difficult.

Furthermore, there can be excessive movement between the bone and the disclosed end rings after implantation. This may delay, and perhaps prevent bone fusion.

Another implant for fusion of bone, particularly vertebrae consisting of a multiplicity of axially mountabte members is known from DE-A-195 09 317 ULRICH. This known implant comprises two terminal implant members and an intermediate third implant member which is rotatable connected about a common axis with the terminal members. The intermediate member is threadably connected with one terminal member, such that the length of the implant is adjustable through rotating the intermediate member. DE-A-19509317 discloses the features of the preamble of claim 1.

As the discussion above illustrates, there is a need for an improved end member for use with a bone fusion implant.

The invention solves this problem by means of a device for fusion of bone, particularly vertebrae, and in particular to an end member for an implant for fusing bone or particularly vertebrae which is characterized by the features of claim 1.

The present invention relates to an end member for use with a bone fusion implant for fusing portions of bone. The end member has a first portion, a second portion sized to be inserted into the bore of the implant, and a shoulder between the first and second portions. When the second portion is inserted into the bore, the shoulder rests on an edge of the implant. The top surface of the first portion conforms in size and shape with the bone and has a channel or multiple channels for receiving a surgical instrument. When multiple channels are present, all the channels can run in the same direction, (e.g. the channels run in the anterolateral direction), or the channels can run in different directions, (e.g. a first channel runs in the anterior-posterior direction and a second channel runs in the lateral direction).

If the implant is made of metal, the end member is made of the same material in order to resist corrosion. The end member may also be made of allograft bone. The end member may have a wedge-shaped or spherical profile to restore the natural curvature of bone or to correct for a non-orthogonal osteotomy. One way to create the wedge-shaped profile is to have the first end of the first portion to be thicker than the second end.

The top surface can be made in any suitable cross-sectional shape, such as oval, oblong, or round, and may be annular or solid. The top surface can be a flat planar surface or can have a curvature that mimics the topography of the bone. The top surface may be provided with teeth or textured for interlocking with the bone. The teeth may have a pyramid or saw tooth shape.

In order to secure the end member to the implant, the second portion comprises a plurality of tabs. These tabs are resilient so that the tabs flex inward upon insertion of the second portion into the implant and flex back outward to create a friction fit that secures the end member to the implant. In another embodiment which is not in accordance with the present invention, the end member includes a projection hinged or pivotably connected to the first portion, a threaded bore in the second portion, and a set screw insertable into the threaded bore. Threading the set screw into the bore causes outward movement of the projection to thereby secure the end member to the implant.

Preferred features of the present invention are disclosed in the accompanying drawings, wherein similar reference characters denote similar elements throughout the several views, and wherein:
FIG. 1 shows a perspective view of a first embodiment of an end member not in accordance with the present invention;
FIG. 2 shows a side view of the end member of FIG. 1 inserted into a cylindrical mesh implant;
FIG. 3 shows a perspective view of an end member having an oblong shape;
FIG. 4a shows a perspective view of an end member having an oblong shape and teeth for improving implant stability;
FIG. 4b shows a perspective view of an end member having first and second channels for facilitating use with implantation instrumentation;
FIG. 4c shows a perspective view of another embodiment of an end member having first and second channels;
FIG. 5 shows a side view of an end member having a wedge-shape profile;
FIG. 6 shows a perspective view of an end member having protuberances for fixation to the implant;
FIG. 7 shows a perspective view of a non-annular end member;
FIG. 8 shows a perspective view of the bottom of an end member according to the present invention;
FIG. 9 shows a perspective view of the bottom of an end member according to the present invention;
FIG. 10 shows a top view of another embodiment of the end member not in accordance with the present invention;
FIG. 11 shows a cross-sectional view of the end member of FIG. 10;
FIG. 12 shows a top view of another embodiment of the end member not in accordance with the present invention;
FIG. 13 shows the end member of FIG. 12 secured to a cylindrical mesh implant; and
FIG. 14 shows a cross-sectional view of another embodiment of the end member not in accordance with the present invention.

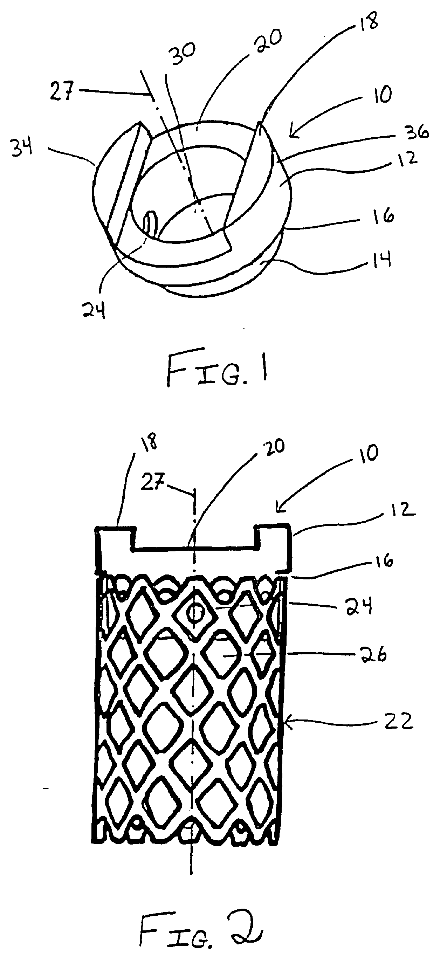

FIG. 1 shows a first embodiment of an end member 10. End member 10 has a first portion 12 and a second portion 14. As first portion 12 is larger than second portion 14, a shoulder 16 is formed at the intersection between first and second portions 12, 14. A top surface 18 of first portion 12 is provided with a first channel 20 for accommodating surgical instrumentation such as holding, insertion, and/or distraction instruments. Top surface 18 is shown in FIG. 1 with a round shape. However, as will be evident from the other embodiments, top surface 18 can have any suitable shape. Preferably, top surface 18 has a shape that matches the shape of the bone it will contact.

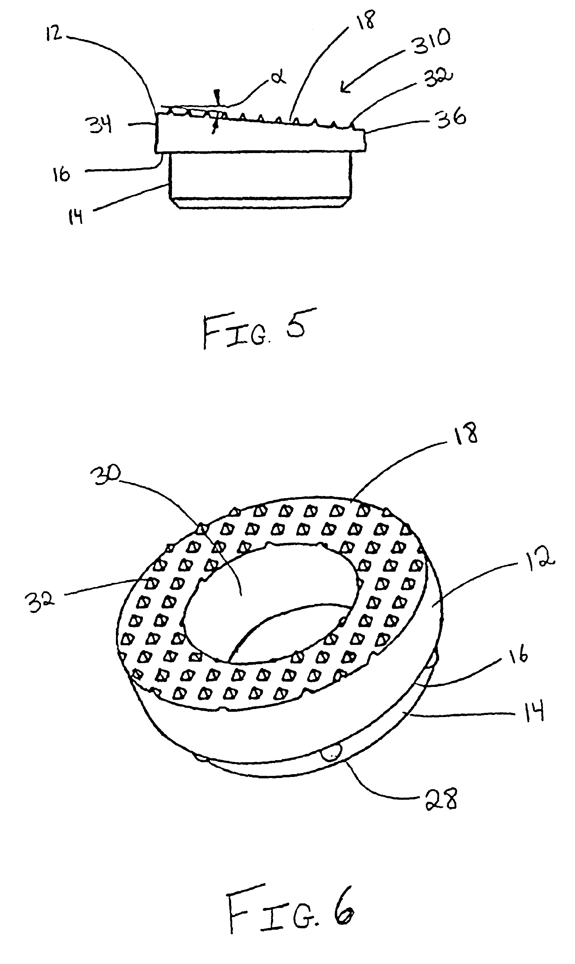

Second portion 14 is also shown having a round shape. As was the case for top surface 18, second portion 14 can have any suitable shape. Preferably, the shape of second portion 14 matches the shape of the bone fusion implant used with the end member. As best seen in FIG. 2, second portion 14 is sized and shaped to be inserted into the inner bore of a bone fusion implant 22 so that shoulder 16 rests on bone fusion implant 22. Bone fusion implant 22 can be a number of different implant types, including, for example, a mesh implant, an allograft implant, or any metallic or non-metallic implant. If implant 22 is made of a metallic material, end member 10 is preferably made of the same metallic material or a non-metallic material to avoid mixed-metal (galvanic) corrosion. End member 10 can also be made of allograft bone from cancellous bone, cortical bone, a combination of cancellous and cortical bone, or a composite of cancellous and cortical bone. After implantation of end member 10 and bone fusion implant 22, physiological compressive forces will tend to hold en member 10 in place against bone fusion implant 22. However, to provide further stability as well as to facilitate pre-operative and intra-operative handling, second portion 14 can be secured to bone fusion implant 22. For example, second portion 14 can be press-fit or snap-fit into bone fusion implant 22. Additionally, because bone fusion implant 22 is typically deformable, bone fusion implant 22 can be crimped to end member 10. Second portion 14 can also be provided with a hole 24 for receiving a fastener such as a pin or a screw. End member 10 is inserted into bone fusion implant 22 so that hole 24 aligns with one of apertures 26 on bone fusion implant 22. Hole 24 can extend either partially or completely through second portion 14 and can be threaded. As shown in FIG. 6, second portion 14 can alternatively have protuberances 28 that are sized to fit in apertures 26 for mechanically securing the end member to the bone fusion implant. Other exemplary mechanisms for securing the end member to the bone fusion implant are described in more detail below.

Referring back to FIGS. 1 and 2, the surface area of top surface 18 is greater than the surface area of the top and bottom surfaces of bone fusion implant 22. Because of this greater surface area, there is more contact area between top surface 18 and the surrounding bone than there would be for the top and bottom surfaces of implant 22. The increase in contact area helps to resist subsidence of implant 22. The increased contact area has other benefits such as greater load sharing between end member 10 and the surrounding bone. Top surface 18 is shown as a ring with an annular space 30. New bone can form in annular space 30 to promote fusion. Annular space can be filled with bone chips or any other osteoinductive or osteoconductive material to promote the formation of bone.

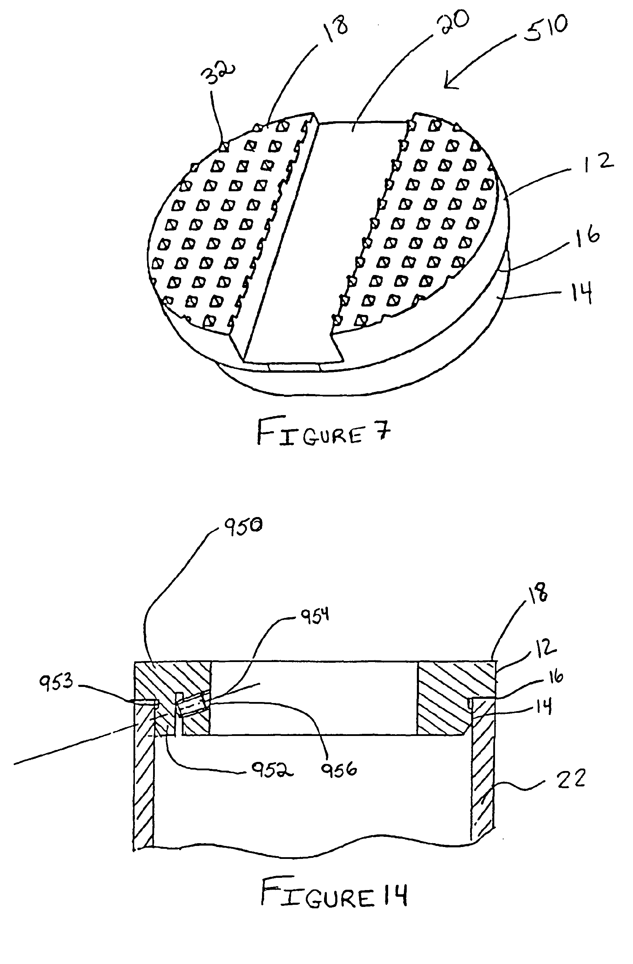

Alternatively, as shown in FIG. 7, top surface 18 can be a solid surface to maximize contact area between top surface 18 and the surrounding bone. A solid top surface 18 would be desirable in clinical situations in which subsidence is especially a concern.

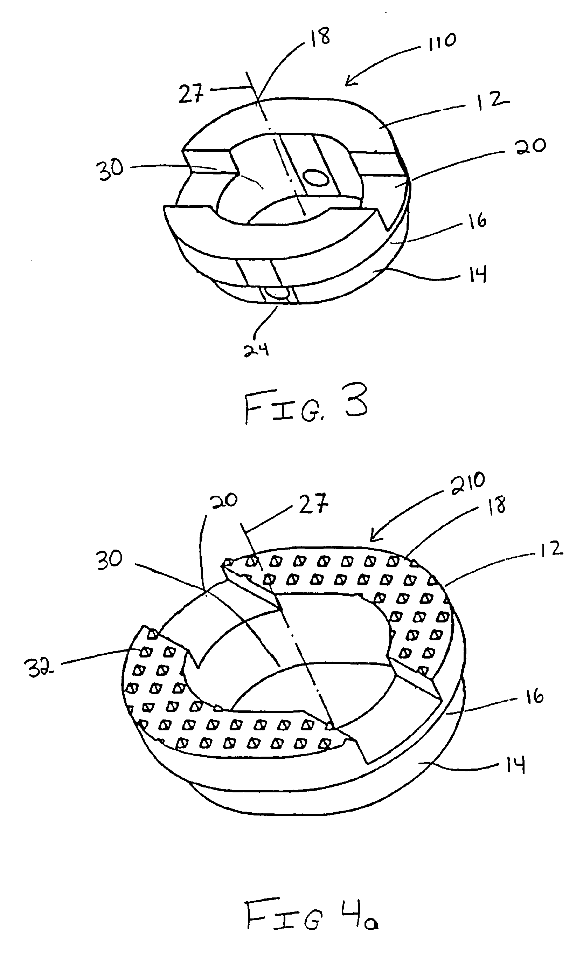

FIG. 3 shows a second embodiment of an end member 110. In general, most of the structure of end member 110 (as well as the embodiments described below) is like or comparable to the structure of end member 10 and, accordingly the same reference numeral is used for like components and discussion of those like components is not believed necessary. End member 110 has an oval or oblong shape and would be used in situations in which the surrounding bone (and consequently the bone fusion implant) is substantially oval or oblong. When the end member has a non-symmetrical shape like end member 110, first channel 20 can be provided in any orientation. For example, first channel 20 is shown running along the long axis of end member 110, but could run in any direction to be oriented differently with respect to the surrounding bone. The variability in the placement of first channel 20 means that first channel 20 can be positioned as best suited for the particular surgical approach that is being used. Specifically, if end member 110 is being implanted with the short axis in the anterior/posterior direction, then first channel 20 is ideal for a lateral surgical approach. If an anterior or posterior approach is anticipated, then first channel 20 should run in the anterior-posterior direction.

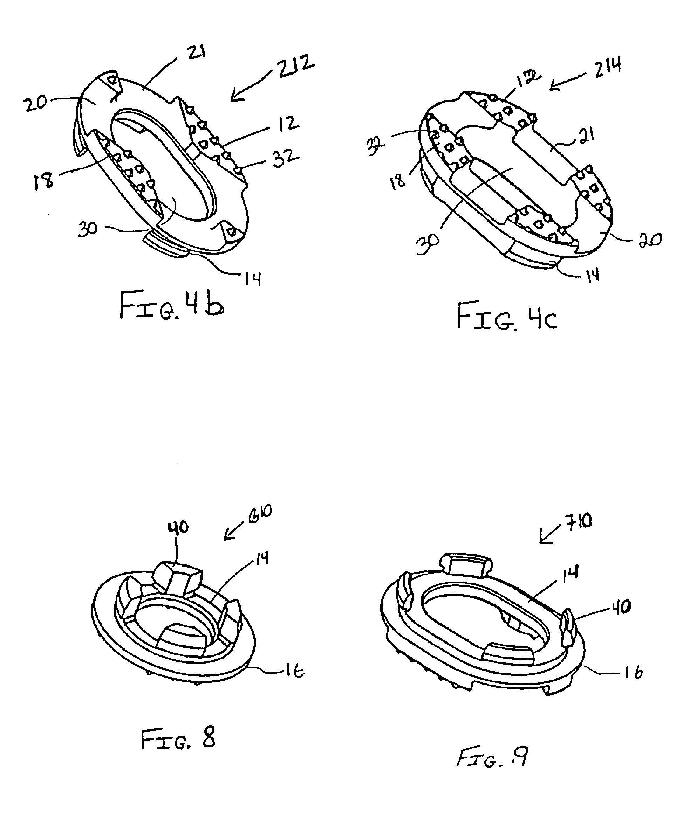

FIG. 4a shows an end member 210 with first channel 20 running at an angle with respect to the long axis of end member 210. This direction of first channel 20 is preferred for an anterolateral surgical approach, which can be used in spinal surgery. FIG. 4b shows an end member 212 that includes first and second channels 20, 21, both running at an angle with respect to the long axis of end member 212. Preferably, first and second channels 20, 21 are symmetrically placed with respect to the long axis of end member 212. Providing end member 212 with both first and second channels 20, 21 allows at least one of first and second channels 20, 21 to be accessed during implantation regardless of whether the left or right side is used for the anterolateral surgical approach. Furthermore, the arrangement of channels 20, 21 allows one end member to be used on each end of bone fusion implant and still have the channels on the top end member align with channels on the bottom end member. FIG. 4c shows an end member 214 that also includes first and second channels 20, 21. First channel 20 runs parallel to the long axis of end member 214 and second channel 21 runs transverse to the long axis of end member 214. Including both first and second channels 20, 21 on end member 214 provides access to at least one of first and second channels 20, 21 regardless of the implantation orientation of end member 214 or the surgical approach.

For each of the end members shown in FIGS. 4a, 4b, and 4c, top surface 18 has a plurality of teeth 32 which provides a mechanical interlock between the end member and the surrounding bone. Teeth 32 provide the mechanical interlock by penetrating the bone. The initial mechanical stability afforded by teeth 32 minimizes the risk of post-operative pullout or expulsion of the end member. Teeth 32 can have any suitable configuration such as pyramid-shaped, saw-tooth shaped, etc. Alternatively, top surface 18 can be textured to provide the mechanical interlock between the end member and the surrounding bone.

As seen in FIG. 5, an end member 310 has a wedge profile. A wedge profile would be useful in spinal applications in order to restore the natural curvature of the spine or any bone. A wedge profile would also be useful to compensate for a non-perpendicular osteotomy cut dictated by the pathology, clinical situation, or erroneously made to remove bone. It should be noted that as an end member would ordinarily be used on each end of bone fusion implant, end members having different wedge profiles can be used together. One way to achieve this wedge shape results from a gradual decrease in thickness in first portion 12 from a first end 34 to a second end 36 so that top surface 18 has a slope defined by angle α. In FIG. 5, top surface 18 is shown as a flat planar surface. However, top surface 18 can be a curved surface and still retain the wedge-shaped profile. As the curve can be made to be a mirror-image of the topography of the vertebral end plates, a curved top surface 18 would conform to the contours of the vertebral end plates.

As previously discussed, FIG. 6 shows an end member 410 that is provided with protuberances 28 sized to fit in apertures 26 of bone fusion implant 22 (FIG. 2). FIG. 7, which has also been discussed, shows an end member 510 having a solid top surface 18 that is preferably used when there is a higher incidence of subsidence.

FIG. 8 shows an end member 610 that has a second portion 14 that includes a plurality of tabs 40 for securing end member 610 to bone fusion implant 22. In accordance with the present invention, tabs 40 are resilient so that as second portion 14 of end member 610 is pushed into bone fusion implant, tabs 40 flex inward and then back outward to secure end member 610 to bone fusion implant 22. As bone fusion implant 22 has some elasticity which would allow it to flex outward to accept tabs 40, tabs 40 need not be resilient. The number, size, and arrangement of tabs 40 can be varied. FIG. 9 shows an end member 710 that is similar to end member 610 except for the overall change in shape (from round to oval or oblong).

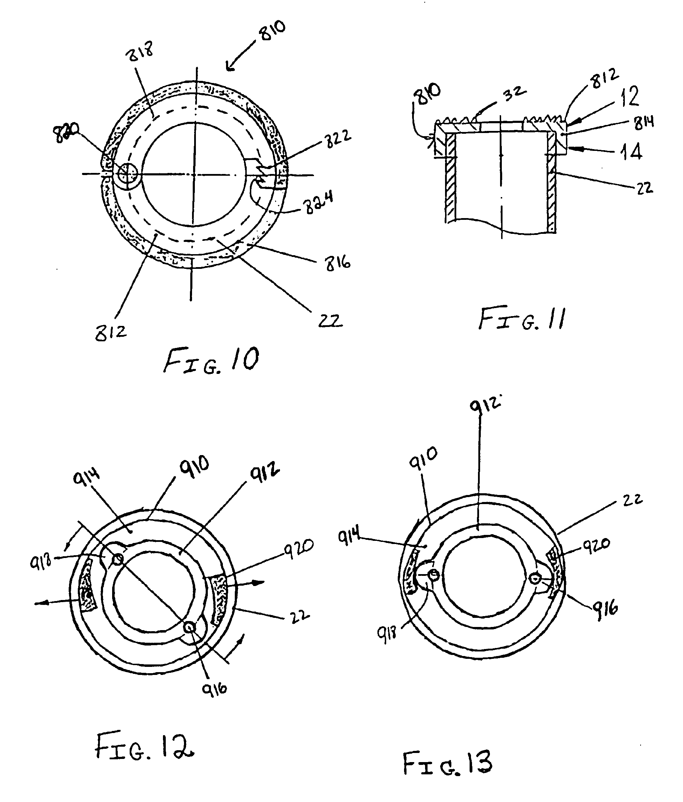

FIGS. 10 and 11 show an end member 810 that has another mechanism for securing end member 810 to bone fusion implant 22. Specifically, end member 810 is a cap that sits on top of implant 22. End member 810 includes a top surface 812 conforming in size and shape with the bone and a sleeve 814 extending from top surface 812. Sleeve 814 is configured and dimensioned to receive a portion of implant 22. In order to facilitate insertion and removal of end member 810 from implant 22, end member 810 is preferably made in two sections 816, 818. In an exemplary embodiment, sections 816, 818 are pivotably connected at a pivot 820 so that sections 816, 818 can be separated, i.e. end member 810 opens. End member 810 also includes a locking mechanism for keeping the two sections 816, 818 in contact. In one embodiment, this locking mechanism comprises a first serrated edge 822 that cooperates with a second serrated edge 824.

FIGS. 12 and 13 show an end member 910 that includes inner and outer rings 912, 914. Inner ring 912 can be rotated relative to outer ring 914. Inner ring 912 has means for receiving a tool to rotate inner ring 912 (shown as holes 916 that accept prongs of the tool). Inner ring 912 also has a pair of protrusions 918 that cooperate with flexible prongs 920 located on outer ring 914. As inner ring 912 is rotated, protrusions 918 contact prongs 920 and force prongs 920 out in a radial direction. When end member 910 is fitted in implant 22, the outward radial movement of prongs 920 causes prongs 920 to press against implant 22 to secure end member 910 to implant 22.

FIG. 14 shows an end member 950 in which second portion 14 includes at least one projection 952. Projection 952 is pivotably connected to second portion 14 at junction 953 so that as a set screw 954 is screwed into threaded bore 956, set screw 954 causes outward movement of projection 952. This outward movement secures end member 950 to implant 22. Junction 953 preferably has a reduced cross section compared to the rest of projection 952 so that the flexing caused by set screw 954 occurs in junction 953.

While various descriptions of the present invention are described above, it should be understood that the various features can be used singly or in any combination thereof.

Therefore, this invention is not to be limited to only the specifically preferred embodiments depicted herein.

Further, it should be understood that variations and modifications within the scope of the invention may occur to those skilled in the art to which the invention pertains. Accordingly, all expedient modifications readily attainable by one versed in the art from the disclosure set forth herein that are within the scope of the present invention are to be included as further embodiments of the present invention. The scope of the present invention is accordingly defined as set forth in the appended claims.

## Claims

1. An end member for use with an implant for fusing bone, particularly vertebrae comprising:
A) a first portion (12) with a longitudinal axis (27) and a top surface (18;812) for contacting a bone, particularly an end plate of a vertebra;
B) a second portion (14) coaxial to the longitudinal axis (27) configured and dimensioned to be mounted at the end of the implant;
C) a shoulder (16) joining the first and second portions (12;14) and sized to rest on an edge of the implant when the second portion (14) is mounted at the implant, whereby
D) the top surface (18) has a first channel (20) for receiving a surgical instrument;
**characterized in that**
E) the second portion (14) comprises a plurality of tabs (40); and
F) each of the plurality of tabs (40) is resilient, flexing inward upon insertion of the second portion (14) in the implant and flexing back outward to secure the end member to the implant.

2. End member of claim 1 wherein the second portion (14) is configured and dimensioned to be inserted into a bore of the implant.

3. End member of claim 1 wherein the second portion (14) comprises a sleeve (814) configured and dimensioned to receive an end of the implant.

4. End member according to one of the claims 1 to 3, wherein the top surface (18;812) has a second channel (21) for receiving a surgical instrument.

5. End member according to one of the claims 1 to 3 wherein the channel (20) penetrates the first portion (12) essentially orthogonal to the longitudinal axis (27).

6. End member of claim 4 or 5 wherein the second channel (21) penetrates the first portion (12) essentially orthogonal to the longitudinal axis (27).

7. End member according to one of the claims 1 to 6 wherein the first channel (20) is open towards the top surface (18;812).

8. End member according to one of the claims 4 to 7 wherein the second channel (21) is open towards the top surface (18;812).

9. End member according to one of the claims 1 to 8 wherein the first channel (20) is configured symmetrical with respect to the longitudinal axis (27).

10. End member according to one of the claims 4 to 9 wherein the second channel (21) is configured symmetrical with respect to the longitudinal axis (27).

11. End member according to one of the claims 1 to 10 wherein the first channel (20) covers between 20% and 80% of the cross-section of the first portion (12).

12. End member according to one of the claims 4 to 10 wherein the second channel (21) covers between 20% and 50% of the cross-section of the first portion (12).

13. End member according to one of the claims 4 to 12 wherein the first channel (20) runs at an angle with respect to the second channel (21).

14. End member according to one of the claims 4 to 12 wherein the first channel (20) runs parallel to the second channel (21).

15. End member according to one of the claims 1 to 14 wherein the top surface (18;812) further comprises a plurality of teeth (32) for interlocking with the bone.

16. End member of claim 15 wherein the teeth (32) have a pyramid shape.

17. End member according to one of the claims 1 to 16 wherein the top surface (18;812) is solid.

18. End member according to one of the claims 1 to 17 wherein the top surface (18;812) is annular.

19. End member according to one of the claims 1 to 17 wherein the top surface (18;812) is circular.

20. End member according to one of the claims 1 to 17 wherein the top surface (18;812) is oval.

21. End member according to one of the claims 1 to 17 wherein the top surface (18;812) is oblong.

22. End member according to one of the claims 1 to 21 wherein the top surface (18;812) is a flat planar surface.

23. End member according to one of the claims 1 to 22 wherein the end member has a wedge-shaped profile.

24. End member of claim 23 wherein the first portion (12) has a first end (34) and a second end (36), the first end height being greater than the second end height to produce the wedge-shaped profile.

25. End member according to one of the claims 1 to 24 wherein the end member is fastenable to the implant.

26. End member of claim 25 wherein the end member is loosenably connectable with the implant.

27. End member of claim 26 wherein the second portion (14) includes a hole (24) for receiving a fastener to secure the end member to the implant.

28. End member of claim 1 or 2 wherein an exterior surface of the second portion (14) has at least one protuberance (28) sized to fit in an aperture of the implant to secure the end member to the implant.

29. End member according to one of the claims 1 to 28 wherein the end member is made of the same material as the implant.

30. End member according to one of the claims 1 to 28 wherein the end member is made of allograft bone.

31. End member of claim 1 or 2 further comprising a projection (952) pivotably connected to the first portion (12), a threaded bore (956) in the second portion (14) and a set screw (954) threadably insertable in the threaded bore, wherein insertion of the set screw (954) in the threaded bore (956) causes outward movement of the projection (952) to secure the end member to the implant.

32. End member according to one of the claims 3 to 31 wherein the first and second portions (12;14) each comprise two sections (816;818) that are pivotable between an open position to facilitate receiving the end of the implant by the sleeve (814) and a closed position to secure the end member to the implant, and a locking mechanism to fix the end member in the closed position.

33. End member of claim 32 wherein the locking mechanism comprises a first serrated edge (822) and a second serrated edge (824) cooperating with each other.

34. End member according to one of the claims 1 to 30 wherein the second portion (14;910) comprises an outer ring (914) having a prong (920) with a ramped surface and an inner ring (912) rotatable with respect to the outer ring (914) and including at least one protrusion (918), the protrusion (918) contacting the ramped surface of the prong (920) upon rotation to thereby urge the prong (920) radially outward.

## Patentansprüche

1. Endstück zur Verwendung mit einem Implantat zur Fusion von Knochen, insbesondere von Wirbelkörpern umfassend:
A) einen ersten Teil (12) mit einer Längsachse (27) und einer für den Knochenkontakt bestimmten Oberseite (18;812), insbesondere einer Endplatte eines Wirbelkörpers;
B) einen zur Längsachse (27) koaxialen zweiten Teil (14), welcher für eine Montage am Ende des Implantates ausgebildet und dimensioniert ist;
C) eine Schulter (16), welche den ersten und zweiten Teil (12;14) verbindet und zur Auflage auf einem Rand des Implantates bemessen ist, wenn der zweite Teil (14) am Implantat montiert ist, wobei
D) die Oberseite (18) einen ersten Kanal (20) zur Aufnahme eines chirurgischen Instrumentes aufweist,
**dadurch gekennzeichnet, dass**
E) der zweite Teil (14) mehrere Nasen (40) umfasst; und
F) jede der mehreren Nasen (40) federnd ist und sich während der Einführung des zweiten Teils (14) in das Implantat einwärts biegen und auswärts zurückbiegen, um das Endstück am Implantat zu sichern.

2. Endstück nach Anspruch 1, **dadurch gekennzeichnet, dass** der zweite Teil (14) für die Einführung in eine Bohrung des Implantates ausgebildet und dimensioniert ist.

3. Endstück nach Anspruch 1, **dadurch gekennzeichnet, dass** der zweite Teil (14) eine Hülse (814) umfasst, welche zur Aufnahme eines Endes des Implantates ausgebildet und dimensioniert ist.

4. Endstück nach einem der Ansprüche 1 bis 3, **dadurch gekennzeichnet, dass** die Oberseite (18;812) einen zweiten Kanal (21) zur Aufnahme eines chirurgischen Instrumentes aufweist.

5. Endstück nach einem der Ansprüche 1 bis 3, **dadurch gekennzeichnet, dass** der Kanal (20) den ersten Teil (12) im wesentlichen orthogonal zur Längsachse (27) durchdringt.

6. Endstück nach Anspruch 4 oder 5, **dadurch gekennzeichnet, dass** der zweite Kanal (21) den ersten Teil (12) im wesentlichen orthogonal zur Längsachse (27) durchdringt.

7. Endstück nach einem der Ansprüche 1 bis 6, **dadurch gekennzeichnet, dass** der erste Kanal (20) gegen die Oberseite (18;812) offen ist.

8. Endstück nach einem der Ansprüche 4 bis 7, **dadurch gekennzeichnet, dass** der zweite Kanal (21) gegen die Oberseite (18;812) offen ist.

9. Endstück nach einem der Ansprüche 1 bis 8, **dadurch gekennzeichnet, dass** der erste Kanal (20) symmetrisch bezüglich der Längsachse (27) ausgebildet ist.

10. Endstück nach einem der Ansprüche 4 bis 9, **dadurch gekennzeichnet, dass** der zweite Kanal (21) symmetrisch bezüglich der Längsachse (27) ausgebildet ist.

11. Endstück nach einem der Ansprüche 1 bis 10, **dadurch gekennzeichnet, dass** der erste Kanal (20) zwischen 20% und 80% des Querschnittes des ersten Teils (12) überdeckt.

12. Endstück nach einem der Ansprüche 4 bis 10, **dadurch gekennzeichnet, dass** der zweite Kanal (21) zwischen 20% und 50% des Querschnittes des ersten Teils (12) überdeckt.

13. Endstück nach einem der Ansprüche 4 bis 12, **dadurch gekennzeichnet, dass** der erste Kanal (20) sich mit einem Winkel zum zweiten Kanal (21) erstreckt.

14. Endstück nach einem der Ansprüche 4 bis 12, **dadurch gekennzeichnet, dass** der erste Kanal (20) sich parallel zum zweiten Kanal (21) erstreckt.

15. Endstück nach einem der Ansprüche 1 bis 14, **dadurch gekennzeichnet, dass** die Oberseite (18;812) zusätzlich mehrere Zähne (32) zur Verblockung mit dem Knochen umfasst.

16. Endstück nach Anspruch 15, **dadurch gekennzeichnet, dass** die Zähne (32) eine Pyramidenform haben.

17. Endstück nach einem der Ansprüche 1 bis 16, **dadurch gekennzeichnet, dass** die Oberseite (18;812) fest ist.

18. Endstück nach einem der Ansprüche 1 bis 17, **dadurch gekennzeichnet, dass** die Oberseite (18;812) ringförmig ist.

19. Endstück nach einem der Ansprüche 1 bis 17, **dadurch gekennzeichnet, dass** die Oberseite (18;812) kreisförmig ist.

20. Endstück nach einem der Ansprüche 1 bis 17, **dadurch gekennzeichnet, dass** die Oberseite (18;812) oval ist.

21. Endstück nach einem der Ansprüche 1 bis 17, **dadurch gekennzeichnet, dass** die Oberseite (18;812) rechteckig ist.

22. Endstück nach einem der Ansprüche 1 bis 21, **dadurch gekennzeichnet, dass** die Oberseite (18;812) eine flache ebene Oberfläche ist.

23. Endstück nach einem der Ansprüche 1 bis 22, **dadurch gekennzeichnet, dass** das Endstück ein keilförmiges Profil hat.

24. Endstück nach Anspruch 23, **dadurch gekennzeichnet, dass** der erste Teil (12) ein erstes Ende (34) und ein zweites Ende (36) hat, wobei die Höhe des ersten Endes grösser als die Höhe des zweiten Endes ist, um das keilförmige Profil herzustellen.

25. Endstück nach einem der Ansprüche 1 bis 24, **dadurch gekennzeichnet, dass** das Endstück an einem Implantat befestigbar ist.

26. Endstück nach Anspruch 25, **dadurch gekennzeichnet, dass** das Endstück lösbar mit dem Implantat verbindbar ist.

27. Endstück nach Anspruch 26, **dadurch gekennzeichnet, dass** der zweite Teil (14) ein Loch (24) zur Aufnahme eines Befestigungselementes zur Fixierung des Endstücks am Implantat umfasst.

28. Endstück nach Anspruch 1 oder 2, **dadurch gekennzeichnet, dass** eine Aussenfläche des zweiten Teils (14) mindestens eine Erhebung (28) hat, welche so bemessen ist, dass sie in eine Öffnung des Implantates passt um das Endstück am Implantat zu sichern.

29. Endstück nach einem der Ansprüche 1 bis 28, **dadurch gekennzeichnet, dass** das Endstück aus demselben Material wie das Implantat hergestellt ist.

30. Endstück nach einem der Ansprüche 1 bis 28, **dadurch gekennzeichnet, dass** das Endstück aus allotransplantiertem Knochen hergestellt ist.

31. Endstück nach Anspruch 1 oder 2, **dadurch gekennzeichnet, dass** das Endstück zusätzlich eine drehbar mit dem ersten Teil (12) verbundene Erhebung (952), eine mit einem Gewinde versehene Bohrung (956) im zweiten Teil (14) und eine in die ein Gewinde aufweisende Bohrung einschraubbare Stellschraube (954) umfasst, wobei das Einführen der Stellschraube (954) in die ein Gewinde aufweisende Bohrung (956) eine Auswärtsbewegung der Erhebung (952) zur Sicherung des Endstücks am Implantat verursacht.

32. Endstück nach einem der Ansprüche 3 bis 31, **dadurch gekennzeichnet, dass** der erste und zweite Teil (12;14) je zwei Abschnitte (816;818), welche zwischen einer offenen Position zur Vereinfachung der Aufnahme des Implantatendes durch die Hülse (814) und einer geschlossenen Position zur Sicherung des Endstücks am Implantat drehbar sind, und einen Blockiermechanismus zur Fixation des Endstücks in der geschlossenen Position umfasst.

33. Endstück nach Anspruch 32, **dadurch gekennzeichnet, dass** der Blockiermechanismus eine erste gezahnte Kante (822) und eine zweite gezahnte Kante (824) umfasst, welche miteinander zusammenwirken.

34. Endstück nach einem der Ansprüche 1 bis 30, **dadurch gekennzeichnet, dass** der zweite Teil (14;910) einen äusseren Ring (914) mit einer Klaue (920) mit einer geneigten Oberfläche und einen inneren Ring (912) umfasst, welcher relativ zum äusseren Ring (914) rotierbar ist und mindestens eine Erhebung (918) umfasst, wobei die Erhebung (918) bei einer Rotation die geneigte Oberfläche der Klaue (920) berührt, um dabei die Klaue (920) radial nach aussen zu pressen.

## Revendications

1. Élément d'extrémité destiné être utilisé avec un implant pour la fusion osseuse, particulièrement des vertèbres, ledit élément d'extrémité comprenant :
A) une première portion (12) présentant à un axe longitudinal (27) et une surface supérieure (18 ; 812) destinée à venir en contact avec un os, notamment une surface articulaire d'une vertèbre ;
B) une seconde portion (14), coaxiale à l'axe longitudinal (27), qui est configurée et dimensionnée pour être montée à l'extrémité de l'implant ;
C) un épaulement (16) raccordant les première et seconde portions (12 ; 14) et dimensionné pour reposer sur un bord de l'implant lorsque la seconde portion (14) est montée sur l'implant,
D) la surface supérieure (18) comportant un premier canal (20) destiné à recevoir un instrument chirurgical ;
**caractérisé en ce que**
E) la seconde portion (14) comprend une pluralité d'ergots (40) ; et
F) chaque ergot de la pluralité d'ergots (40) est élastique, en s'infléchissant vers l'intérieur lors de l'insertion de la seconde portion (14) dans l'implant et en s'infléchissant de nouveau vers l'extérieur pour immobiliser l'élément d'extrémité par rapport à l'implant.

2. Élément d'extrémité selon la revendication 1, dans lequel la seconde portion (14) est configurée et dimensionnée pour être insérée dans un perçage de l'implant.

3. Élément d'extrémité selon la revendication 1, dans lequel la seconde portion (14) comprend un manchon (814) configuré et dimensionné pour recevoir une extrémité de l'implant.

4. Élément d'extrémité selon l'une des revendications 1 à 3, dans lequel la surface supérieure (18 ; 812) possède un second canal (21) destiné à recevoir un instrument chirurgical.

5. Élément d'extrémité selon l'une des revendications 1 à 3, dans lequel le canal (20) pénètre dans la première portion (12) sensiblement orthogonalement à l'axe longitudinal (27).

6. Élément d'extrémité selon la revendication 4 ou 5, dans lequel le second canal (21) pénètre dans la première portion (12) sensiblement orthogonalement à l'axe longitudinal (27).

7. Élément d'extrémité selon l'une des revendications 1 à 6, dans lequel le premier canal (20) est ouvert en direction de la surface supérieure (18 ; 812).

8. Élément d'extrémité selon l'une des revendications 4 à 7, dans lequel le second canal (21) est ouvert en direction de la surface supérieure (18 ; 812).

9. Élément d'extrémité selon l'une des revendications 1 à 8, dans lequel le premier canal (20) est configuré de façon symétrique par rapport à l'axe longitudinal (27).

10. Élément d'extrémité selon l'une des revendications 4 à 9, dans lequel le second canal (21) est configuré de façon symétrique par rapport à l'axe longitudinal (27).

11. Élément d'extrémité selon l'une des revendications 1 à 10, dans lequel le premier canal (20) couvre entre 20% et 80% de la section transversale de la première portion (12).

12. Élément d'extrémité selon l'une des revendications 4 à 10, dans lequel le second canal (21) couvre entre 20% et 50% de la section transversale de la première portion (12).

13. Élément d'extrémité selon l'une des revendications 4 à 12, dans lequel le premier canal (20) s'étend en formant un angle par rapport au second canal (21).

14. Élément d'extrémité selon l'une des revendications 4 à 12, dans lequel le premier canal (20) s'étend parallèlement au second canal (21).

15. Élément d'extrémité selon l'une des revendications 1 à 14, dans lequel la surface supérieure (18 ; 812) comprend en outre une pluralité de dents (32) destinées à s'engager avec l'os.

16. Élément d'extrémité selon la revendication 15, dans lequel les dents (32) ont une forme pyramidale.

17. Élément d'extrémité selon l'une des revendications 1 à 16, dans lequel la surface supérieure (18 ; 812) est pleine.

18. Élément d'extrémité selon l'une des revendications 1 à 17, dans lequel la surface supérieure (18 ; 812) est annulaire.

19. Élément d'extrémité selon l'une des revendications 1 à 17, dans lequel la surface supérieure (18 ; 812) est circulaire.

20. Élément d'extrémité selon l'une des revendications 1 à 17, dans lequel la surface supérieure (18 ; 812) est ovale.

21. Élément d'extrémité selon l'une des revendications 1 à 17, dans lequel la surface supérieure (18 ; 812) est oblongue.

22. Élément d'extrémité selon l'une des revendications 1 à 21, dans lequel la surface supérieure (18 ; 812) est une surface plane plate.

23. Élément d'extrémité selon l'une des revendications 1 à 22, dans lequel l'élément d'extrémité a un profil cunéiforme.

24. Élément d'extrémité selon la revendication 23, dans lequel la première portion (12) possède une première extrémité (34) et une seconde extrémité (36), la hauteur de la première extrémité étant supérieure à la hauteur de la seconde extrémité pour générer le profil cunéiforme.

25. Élément d'extrémité selon l'une des revendications 1 à 24, dans lequel l'élément d'extrémité peut être fixé à l'implant.

26. Élément d'extrémité selon la revendication 25, dans lequel l'élément d'extrémité peut être raccordé de façon amovible à l'implant.

27. Élément d'extrémité selon la revendication 26, dans lequel la seconde portion (14) inclut un trou (24) destiné à recevoir une fixation pour immobiliser l'élément d'extrémité à l'implant.

28. Élément d'extrémité selon la revendication 1 ou 2, dans lequel une surface extérieure de la seconde portion (14) possède au moins une protubérance (28) dimensionnée pour s'insérer dans une ouverture de l'implant afin d'immobiliser l'élément d'extrémité par rapport à l'implant.

29. Élément extrémité selon l'une des revendications 1 à 28, dans lequel l'élément d'extrémité est fabriqué à partir du même matériau que l'implant.

30. Élément d'extrémité selon l'une des revendications 1 à 28, dans lequel l'élément d'extrémité est fabriqué à partir d'une allogreffe osseuse.

31. Élément d'extrémité selon la revendication 1 ou 2 comprenant en outre une projection (952) raccordée, de façon à pouvoir pivoter, à la première portion (12), un perçage fileté (956) ménagé dans la seconde portion (14) et une vis sans tête (954) pouvant être insérée par vissage dans le perçage fileté, l'insertion de la vis sans tête (954) dans le perçage fileté (956) causant un mouvement de la projection (952) vers l'extérieur afin d'immobiliser l'élément d'extrémité par rapport à l'implant.

32. Élément d'extrémité selon l'une des revendications 3 à 31, dans lequel les première et seconde portions (12 ; 14) comprennent chacune deux sections (816 ; 818) qui sont aptes à pivoter entre une position ouverte pour faciliter la réception de l'extrémité de l'implant par le manchon (814) et une position fermée pour immobiliser l'élément d'extrémité par rapport à l'implant, et un mécanisme de verrouillage pour fixer l'élément d'extrémité dans la position fermée.

33. Élément d'extrémité selon la revendication 32, dans lequel le mécanisme de verrouillage comprend un premier bord strié (822) et un second bord strié (824) coopérant l'un avec l'autre.

34. Élément d'extrémité selon l'une des revendications 1 à 30, dans lequel la seconde portion (14 ; 910) comprend une bague extérieure (914) possédant une griffe (920) dotée d'une surface inclinée et une bague intérieure (912) apte à tourner par rapport à la bague extérieure (914) et incluant au moins une saillie (918), la saillie (918) venant en contact avec la surface inclinée de la griffe (920) lors d'une rotation afin de pousser la griffe (920) radialement vers l'extérieur.
